# EUROPEAN PATENT SPECIFICATION

(11) **EP 2 190 390 B1**
(45) Date of publication and mention of the grant of the patent: **30.12.2020**
(21) Application number: 08797282.4
(22) Date of filing: 06.08.2008
(51) Int. Cl.: A61F 9/02

(54) **GOGGLE LENS ATTACHMENT SYSTEM AND METHOD**
SYSTEM UND VERFAHREN ZUR BEFESTIGUNG VON BRILLENGLÄSERN
SYSTÈME ET PROCÉDÉ DE FIXATION DE LUNETTES PROTECTRICES

(30) Priority: 09.08.2007 US 836729
(43) Date of publication of application: 02.06.2010
(73) Proprietor: Smith Optics, Inc., Ketchum ID 83340 (US)
(72) Inventor: MCNEAL, Joseph, R., Hailey, Idaho 83333 (US); GIROUX, George, T., Ketchum, Idaho 83340 (US)
(74) Representative: Ridley, Daniel
(86) International application number: PCT/US2008/072332
(87) International publication number: WO 2009/023491

(56) References cited:
- DE-U1- 20 109 028
- US-A- 3 383 707
- US-A- 3 945 044
- US-A- 5 802 622
- US-A- 6 047 410
- US-A1- 2007 033 718
- US-A1- 2007 153 230
- US-B1- 6 349 420
- US-B1- 6 637 877

## Description

This invention relates generally to eyewear, such as ski goggles, and more specifically to methods and apparatus for removably securing a lens to a goggle frame.

Goggles are widely used in a variety of applications, such as when skiing and snowboarding, working in hazardous conditions or with hazardous substances. Goggles typically include a frame having a somewhat oval configuration with an inner edge adapted to contact a wearer's face, often through a cushioning foam face gasket. The frame usually has an arcuate shape curving rearwardly at the sides to conform to a wearer's face. A lens is normally mounted in the frame near or at an outer edge of the frame so that the lens and wearer's face substantially enclose the interior of the frame. The goggle is normally held on the wearer's head by an elastic headband having its ends attached to opposite side edges of the goggle frame. The headband may be attached directly to the goggle frame, or it may be attached through an intervening structure.

The lenses of modern goggles are often tinted to shield the wearer's eyes from the glare from the snow. However, the level of light reduction needed depends on weather conditions and the time of day. In cloudy conditions less glare reduction is needed and the wavelengths that need to be filtered by the lens may be different. Likewise, when night skiing it may be desirable to have no tinting whatsoever.

The lens provided with goggles may also become inadequate as they are scratched or otherwise damaged. For example, some lenses have metallicized coatings that are easily scratched.

In view of the foregoing it would be an advancement in the art to provide eyewear, particularly a ski goggle having a readily replaceable lens in order to enable a user to use different lenses having an appropriate level of glare reduction without the need for multiple goggles. Such a replaceable lens would further enable replacement of damaged lenses without the need to purchase an additional goggle frame.

US 2007/0153230 A discloses an eyewear system which includes a frame having a lens channel configured to slidably receive the lens and an opening to allow the lens to be inserted into the lens channel. The system further includes a lens configured to be slidably received into the lens channel of the frame and a latch to releasably secure the lens in the frame. More specifically, the eyewear system includes a lens, a top rail attached to the lens, and a frame that includes a lens support. The lens support includes a lens channel for receiving the lens. In some embodiments, the lens includes snap features along the perimeter that engage the lens channel. The system may also include outriggers that may retain a strap. In some embodiments, the outriggers secure the lens to the frame. This is achieved by inserting posts of the outriggers into holes in the top rail and/or the frame. In other embodiments the outriggers are attached to the top rail and/or frame with a latch. The outriggers engage the top rail and/or the frame. In other embodiments, a latch is mounted to the frame, such as on the lens support. The latch engages the top rail to secure the lens to the frame.

US 3 383 707 A discloses a flip-up sunglass construction comprising a pair of gripping fingers on each side of a nose piece with a pintle on a lens sheet snap-engaged between the fingers, whereby the lens sheet is mounted by said pintle and fingers for swinging movement relative to a frontpiece extending along the brow of a user.

The invention relates to an eyewear according to claim 1. Embodiments are disclosed in the dependent claims.

In addition, following aspects are disclosed:

A goggle includes a lens having right and left eye portions. A frame is selectively engaged with a perimeter of the lens and includes an upper portion extending across an upper perimeter of the right and left eye portions and a lower portion extending across a lower perimeter of the right and left eye 'portions. One of the upper and lower portions is substantially more rigid than the other of the upper and lower portions and has at least one fastener secured thereto. The fastener selectively engages the lens to retain the lens in engagement with the frame.

In another aspect, the fastener includes a latch pivotally coupled to one of the upper and lower frame portions. The latch is selectively pivotable between a first position in which the latch retains the lens with respect to the frame and a second position in which the latch permits the lens to be removed from the frame.

In another aspect, the lens defines a slot extending therethrough. The latch includes an elongate member mounted on a shaft secured to the frame. The elongate member is rotatable within the slot to a crosswise position in which the slot interferes with removal of the elongate member from the slot.

In another aspect a plate secures to the elongate member having a portion of the lens positioned between the plate and the frame when the latch is in the first position.

In another aspect, an over-center mechanism engages the latch, the over-center mechanism biases the latch away from an intermediate position between the first and second positions and resists movement of the latch between the first and second positions.

In another aspect strap mounts are secured to the frame, each having first and second ends and an intermediate portion extending between the first and second ends. The first ends secure to the upper frame portion and the lower ends secure to the lower frame portion. The strap secures to the intermediate portion such that the intermediate portion urges the lens against the frame upon tensioning of the strap.

### BRIEF DESCRIPTION OF THE DRAWINGS

Figure 1 is an isometric view of a goggle having a removable lens in accordance with an embodiment of the present invention.
Figure 2 is a front plan view of a removable lens in accordance with an embodiment of the present invention.
Figures 3A and 3B are isometric views of a latching mechanism for securing the removable lens to the lower portion of a goggle frame in accordance with an embodiment of the present invention.
Figure 4 is a front elevation view of a frame suitable for receiving a removable lens in accordance with an embodiment of the present invention.
Figure 5 is an isometric view of a pivoting latch in accordance with an embodiment of the present invention.
Figure 6 is an isometric view of a seat for receiving a pivoting latch in accordance with an embodiment of the present invention.
Figures 7A and 7B are cross-sectional views showing an over-center mechanism suitable for use with the pivoting latch of Figure 6 in accordance with an embodiment of the present invention.
Figure 8A and 8B are side cross-sectional views of a fastener for securing the removable lens to the upper portion of the frame in accordance with an embodiment of the present invention.
Figure 9 is a top plan view of a frame and removable lens having pivoting strap mounts in accordance with an embodiment of the present invention.
Figure 10 is an isometric view of a pivoting strap mount in accordance with an embodiment of the present invention.

### DETAILED DESCRIPTION OF PREFERRED EMBODIMENTS

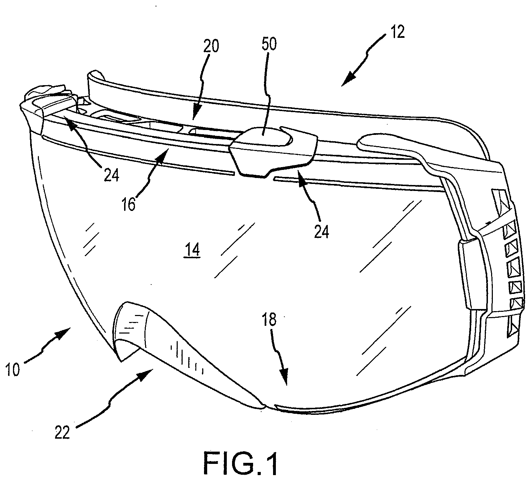

Referring to Figure 1, a goggle 10 includes a frame 12 and a lens 14 secured to the frame 12. The lens 14 includes an upper perimeter 16 and a lower perimeter 18 engaging an upper portion 20 and lower portion 22, respectively, of the frame. One or more fasteners 24 are secured to one of the upper and lower portions 20, 22 for securing the lens 14 to the frame 12. For example, in the illustrated embodiment, the fasteners 24 are secured to the upper portion 20 of the frame 12 which is substantially more rigid than the lower portion 22. The fasteners 24 are preferably manually releasable to enable ready assembly and disassembly of the lens 14 and frame 12. The portion 20, 22 to which the fastener 24 secures may be relatively rigid as compared to the other portion 20, 22. In some embodiments, a stiffening member is provided within the more rigid portion 20, 22. The stiffening member may be fastened to the more rigid portion 20, 22, or be integrally or monolithically formed with the more rigid portion 20, 22.

In an alternative embodiment, the frame portions 20, 22 may be of the same or dissimilar stiffness. In such embodiments, four fasteners 24 may be used, two mounted to the upper portion 20 and two mounted to the lower portion 22. In other alternative embodiments, the goggle 10 is replaced by sunglasses having two lenses 14 mounted to the frame 12. The sunglasses may include two fasteners 24 one selectively engaging each lens near the top, bottom, or sides, of the lens. Alternatively, each lens may be inserted in the frame and selectively engage two fasteners 24 mounted to the frame, one at the top and bottom, or one either side, of each lens.

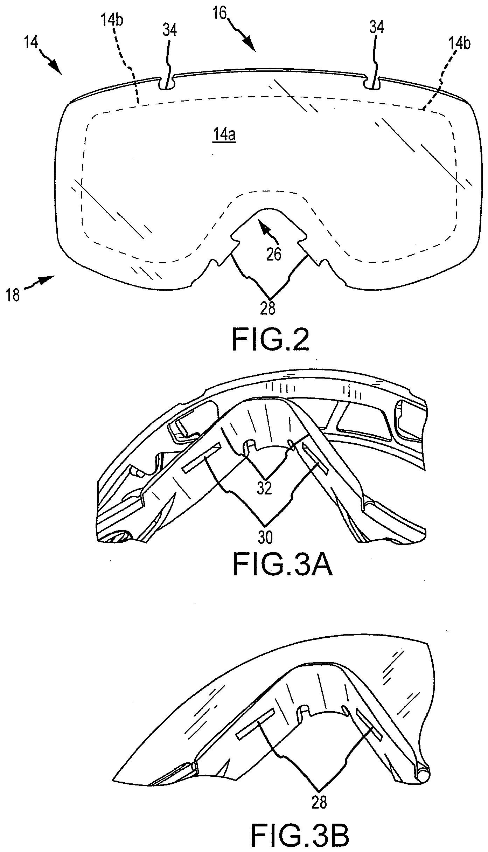

Referring to Figure 2, a notch 26 for receiving the wearer's nose is formed in the lower perimeter 18 of the lens 14 between right and left eye portions of the lens 14. Tabs 28 are formed on opposing faces of the notch 26 for removably securing the lens 14 to the lower portion 22 of the frame 12.

Referring to Figures 3A and 3B, while still referring to Figures 1 and 2, the tabs 28 are inserted within slots 30 formed in the lower portion 22. Specifically, the slots 30 are formed in angled members 32 disposed to be supported by the nose of the wearer. The angled members 32 are typically flexible and/or flexibly secured to one another or to the rest of the frame such that the angled members 32 may deflect to allow the tabs 28 to be inserted within the slots 30 and removed from the slots 30. In an alternative embodiment, tabs formed on the angled members 32 engage slots or recesses formed in the lower perimeter 18 of the lens 14. Other fastening means may also be used.

Referring again to Figure 2, slots 34 may be formed in the upper perimeter 16 of the lens 14 for engaging the fastener 24. In other embodiments, slots 34 are formed in the lower perimeter 18, or in both the upper and lower perimeters 16, 18 and engage fasteners 24 mounted on the upper and lower frame portions 20, 22 that have the same or dissimilar stiffness. In one method of use, the lower perimeter 18 is secured to the lower portion 22 of the frame 12. The lower portion 22 of the frame 12 with the lens 14 attached is then deflected away from the upper portion 20 of the frame 12 in order to position the slots 34 below the fastener 24. The lower portion 22 and lens 14 are then deflected toward the upper portion 20, which brings the slots 34 into engagement with the fastener 24. The fastener 24 may then be transitioned to a locked state in which the upper perimeter 16 of the lens 14 is retained against the upper portion 20 of the frame 12.

In some embodiments, the lens 14 includes a first lens 14a and a second lens 14b defining a closed volume between them to provide insulation between the outside air and air trapped between the goggle 10 and the wearer's face. In such embodiments, the second lens 14b may be smaller than the first lens 14a such that the slots 34 are formed in a portion of the second lens extending outwardly from the perimeter of the second lens 14b. The tabs 28 may likewise be formed in a portion of the first lens 14a extending outwardly from the perimeter of the second lens 14b.

In the illustrated embodiment, the second lens 14b is sized to be inserted within the frame 12 whereas the rear surface of the first lens 14a is pressed against the face of the frame 12, with the exception of the notch 26, which may be received by grooves formed in the angled members 32, as shown in Figure 1.

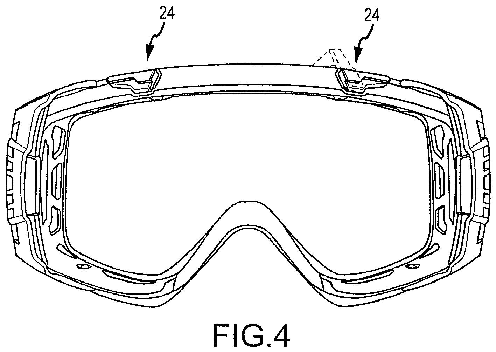

Referring to Figure 4, in the illustrated embodiment the fastener 24 is pivotally secured to the upper portion 20 of the frame 12. The fastener 24 may have an open position, shown in dotted lines, wherein the slots 34 can be moved freely into and out of engagement with the fastener. The fastener 24 also has a closed position wherein the lens 14 is retained by the fastener.

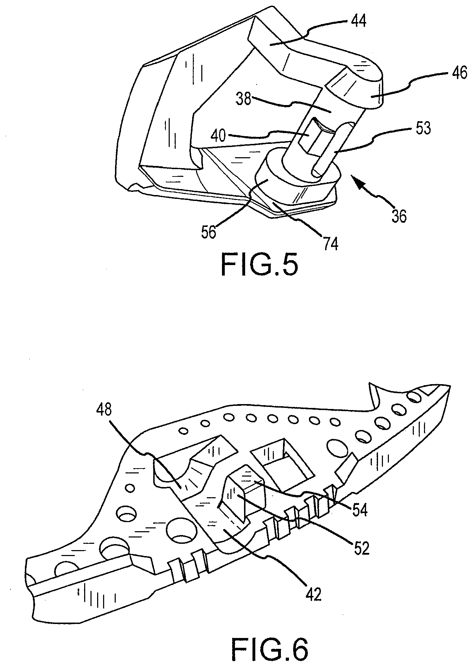

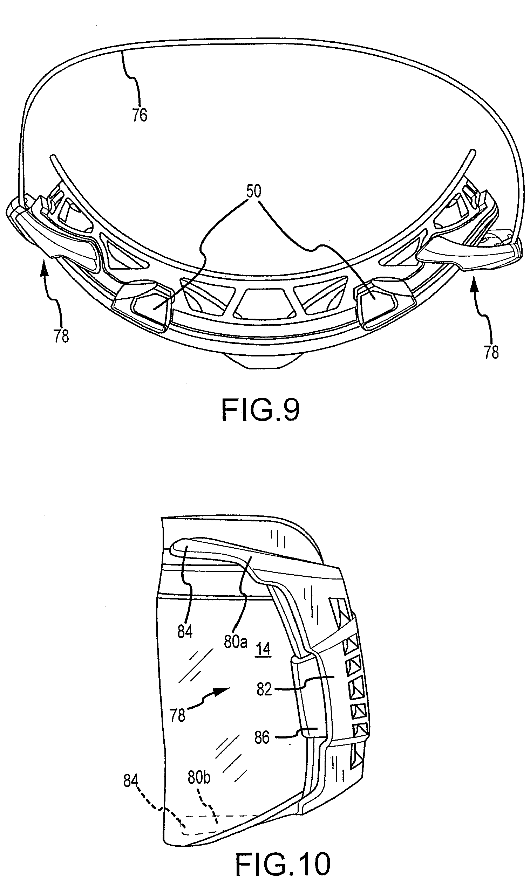

Referring to Figures 5 and 6, in some embodiments the fasteners 24 each have an over-center mechanism 36 such that the fastener 24 is biased away from an intermediate position between the open and closed positions. In the illustrated embodiment, the over-center mechanism 36 includes a shaft 38 bearing a projection 40. Other over-center mechanisms 36 known in the art may also be used. The illustrated shaft 38 is pivotally mounted to the frame, such as by means of a seat 42 formed on or secured to the frame. The shaft 38 may be secured to an arm 44 enabling a user to rotate the shaft 38. In the illustrated embodiment, a cap 46 is secured to the shaft 38 and fits within a recess 48 such that the shaft 38 resists removal along the longitudinal axis of the shaft. The shaft 38 may be retained within the seat 42 by a retaining member 50 fastened to the frame 12 over the seat 42. (See Figures 1 and 9).

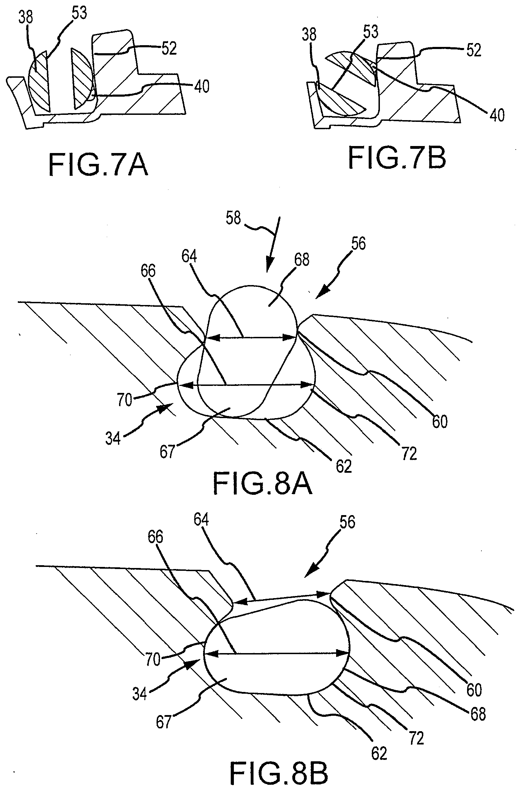

Referring to Figures 7A and 7B, while still referring to Figures 5 and 6, the projection 40 engages a surface 52 fixedly secured to the frame 12. In the illustrated embodiment, the surface 52 is formed on a post 54 extending upwardly from the seat 42.

As shown in Figure 7A, in one position, the projection 40 is located to one side of the point where the shaft 38 and surface 52 are closest to one another, whereas in Figure 7B, the projection is located on the opposite side. In order to move between the orientations of Figures 7A and 7B, the projection 40 must move across the surface 52. This requires either the surface 52 or shaft 38 to resiliently deflect, providing the biasing force urging the shaft 38 toward either the open or closed position. In the illustrated embodiment, the shaft 38 includes a slot 53 facilitating bending of the shaft 38 when transitioning between the open and closed positions.

Referring to Figures 8A and 8B, while still referring to Figures 5 and 6, the fasteners 24 may each include an elongate member 56 secured to the shaft 38 having its direction of elongation perpendicular to the axis of rotation and/or longitudinal axis of the shaft. In the open position, the elongate member 56 is oriented relative to the slot 34 such that the elongate member 56 may be inserted along an insertion direction 58 through an opening 60 of the slot 34 that extends from the perimeter of the lens 14 to a widened portion 62 having a width 64 substantially greater than the width 66 of the opening. The width 64 is typically such that the elongate member 56 may be rotated into the closed position shown in Figure 8B within the widened portion 62.

In some embodiments, the elongate member 56 is formed of a resilient material such that the width 64 is smaller than the length of the elongate member 56 along its direction of elongation and must therefore be deformed to achieve the orientation of Figure 8B. As is apparent in Figure 8B, in the closed position the slot 34 interferes with movement of the elongate member 56 along the insertion direction to a greater extent than in the open position shown in Figure 8A. In some embodiments, the elongate member 56 must deform in order to transition from the open position to the closed position. In the illustrated embodiment, the elongate member 56 includes a narrow end 67 and widened end 68. In such embodiments, the slot 34 may likewise include a narrow end 70 and a widened end 72. In the illustrated embodiment, the narrow end 67 is inserted first. As the elongate member 56 is rotated, the narrow end 67 draws the lens 14 upward toward the fastener 24 and the upper portion 20. The widened end 68 slides along the slot 34, deforming slightly, and then seats in the widened end 72 of the slot 34. The deformation required to remove the elongate member 56 from the slot 34 thereafter serves to retain it in the slot 34. The over-center mechanism 36 also hinders rotation of the elongate member 56 out of the slot 34.

Referring again to Figure 4, while still referring to Figure 8A and 8B, in some embodiments, the elongate members 56 of the two fasteners 24 have opposite orientations such that they rotated in opposite directions when moving between the open and closed position. Accordingly, the arms 44 secured to the shafts 38 may be mirrored as illustrated in Figure 4.

Referring again to Figures 5 and 6, the engagement of the elongate member 56 with the slot 34 may cooperate with a plate 74 to fasten the lens 14 to the frame. The plate 74 is secured to the shaft 38 or the elongate member 56 and captures the lens between itself and the frame 12 in order to prevent the slot 34 from sliding off the elongate member 56. In alternative embodiments, the plate 74 is not secured to the shaft 38 or elongate member 56, but rather is fixedly secured to the frame 12.

In one method of use, the lens 14 is first secured to the lower frame portion 22 as discussed above. The lower frame portion 22 and lens 14 are then moved away from the upper frame portion 20 by deflecting the lower frame portion 22 until the upper perimeter 16 of the lens 14 is positioned below the plates 74. The lens 14 is then moved upward between the plates 74 and the upper frame portion 20 and the elongate members 56 are inserted within the slots 34. The elongate members 56 are then rotated within the slot 34 as illustrated in Figures 8A and 8B to secure the upper perimeter 16 of the lens 14 to the upper frame portion 20.

Referring to Figures 9 and 10, the goggle 10 includes a strap 76 for encircling the head of the wearer in order to retain the frame 12 against the face of the wearer. The strap 76 is preferably formed of an elastic material and is under tension when the goggle 10 is worn. The strap 76 may have an adjustable length.

The strap 76 is secured to the goggle 10 by means of strap mounts 78 secured on the right and left sides of the goggle. In some embodiments, the strap mounts 78 are pivotally mounted to the frame 12 and facilitate wearing of the goggle 10 by an individual wearing a helmet. For example, the strap mounts 78 may include arms 80a, 80b secured to the upper and lower frame portions 20, 22, respectively. An intermediate portion 82 extends between the arms 80a, 80b. The arms 80a, 80b pivotally secure to the upper and lower frame portions 20, 22, respectively, at points of securement 84 located inwardly from the right and left ends of the goggle 10. The intermediate portions 82 of the strap mounts 78 are located closer to the right and left ends, respectively, of the goggle 10 than the points of securement 84 such that when the strap 76 is secured to the intermediate portion 82 and tensioned the intermediate portion is urged against the lens 14. A pad 86 may be secured to the intermediate portion 82 such that the pad 86 is positioned between the intermediate portion 82 and the lens 14. The pad 86 is preferably formed of a resilient material such as a natural or synthetic rubber.

In use, the intermediate portion 82 is pivoted away from the frame 12 when the lens is inserted. After the lens 14 is positioned against the frame, the intermediate portion 82 is pivoted toward the lens and may serve to urge the lens 14 against the frame 12 when tension is applied to the strap 76.

From the foregoing it will be appreciated that, although specific embodiments of the invention have been described herein for purposes of illustration, various modifications may be made without deviating from the spirit and scope of the invention. Accordingly, the invention is not limited except as by the appended claims.

## Claims

1. Eyewear (10) comprising:
a lens (14) having an upper perimeter (16) and a lower perimeter (18), the lens defining at least one slot (34) formed in one of the upper and lower perimeters (16, 18);
a frame (12) selectively engaging a perimeter of the lens (14), the frame comprising an upper portion (20) extending across the upper perimeter (16) and a lower portion (22) extending across the lower perimeter (18); and
a fastener (24), pivotally mounted to one of the upper and lower portions (20, 22), the fastener (24) selectively engaging the at least one slot (34) to retain the lens (14) in engagement with the frame (12),
wherein the fastener (24) includes an elongate member (56) mounted on a shaft (38) rotatably mounted to the frame (12) and having its direction of elongation perpendicular to the axis of rotation of the shaft (38), the elongate member (56) rotatable within the slot (34) from an open position to a closed position, wherein in the open position the elongate member (56) is oriented relative to the slot (34) such that the elongate member (56) is configured to be inserted along an insertion direction (58) through an opening (60) of the slot (34) that extends from the perimeter of the lens (14) to a widened portion (62) having a width (64) greater than the width (66) of the opening, and in the closed position the slot (34) interferes with movement of the elongate member (56) along the insertion direction (58) to a greater extent than in the open position.

2. The eyewear of claim 1, wherein the elongate member (56) is insertable through the opening (60) in the open position perpendicular to the closed position and is not insertable through the opening (60) in the closed position.

3. The eyewear of claim 2, further comprising an over-center mechanism (36) engaging the shaft (38), the over-center mechanism (36) biasing the shaft (38) and elongate member (56) away from an intermediate position between the open and closed positions.

4. The eyewear of claim 2, further comprising a plate (74) secured to the elongate member (56), a portion of the lens (14) positioned between the plate (74) and the frame (12) when the elongate member (56) is in the closed position within the slot (34).

5. The eyewear of claim 1, wherein the elongate member (56) has first and second ends (67, 68), the second end (68) being wider than the first end (67).

6. The eyewear of claim 1, wherein the lens (14) includes first and second lenses (14a, 14b) secured to one another, the slot (34) being formed in a portion of the second lens (14b) extending outwardly from a perimeter of the first lens (14a).

7. The eyewear of claim 6, wherein the perimeter of the first lens (14a) is sized to fit within an inner perimeter of the frame (12) and wherein the second lens (14b) engages a face of the frame (12) extending outwardly from the inner perimeter.

8. The eyewear of claim 1, wherein the eyewear is a goggle (10).

9. The eyewear of claim 1 , wherein the eyewear is sunglasses.

10. The eyewear of claim 1, wherein the slot (34) is a first slot positioned in the upper perimeter (16) of the lens (14) and wherein the fastener (24) is a first fastener mounted to the upper portion (20) of the frame (12), the eyewear further comprising a second slot (34) positioned in the lower perimeter (18) of the lens (14) and a second fastener (24) mounted to the lower portion (22) of the frame (12), the second fastener (24) selectively engaging the second slot (34) to retain the lens (14) in engagement with the frame (12).

## Patentansprüche

1. Brille (10), umfassend:
eine Linse (14) mit einem oberen Rand (16) und einem unteren Rand (18), wobei die Linse mindestens einen Schlitz (34) definiert, der in dem oberen oder dem unteren Rand (16, 18) ausgebildet ist,
eine Fassung (12), die einen Rand der Linse (14) gezielt in Eingriff nimmt, wobei die Fassung einen oberen Abschnitt (20), der sich über den oberen Rand (16) erstreckt, und einen unteren Abschnitt (22), der sich über den unteren Rand (18) erstreckt, umfasst, und
ein Befestigungselement (24), das schwenkbar an dem oberen oder dem unteren Abschnitt (20, 22) montiert ist, wobei das Befestigungselement (24) den mindestens einen Schlitz (34) gezielt in Eingriff nimmt, um die Linse (14) in Eingriff mit der Fassung (12) zu halten,
wobei das Befestigungselement (24) ein längliches Glied (56) aufweist, das an einem drehbar an der Fassung (12) montierten Schaft (38) montiert ist und dessen Hauptausdehnung senkrecht zu der Rotationsachse des Schafts (38) verläuft, wobei das längliche Glied (56) in dem Schlitz (34) aus einer offenen Position in eine geschlossene Position drehbar ist, wobei das längliche Glied (56) in der offenen Position so bezüglich des Schlitzes (34) ausgerichtet ist, dass das längliche Glied (56) dazu ausgestaltet ist, entlang einer Einführrichtung (58) durch eine Öffnung (60) des Schlitzes (34) eingeführt zu werden, der sich von dem Rand der Linse (14) zu einem verbreiterten Abschnitt (62) mit einer Breite (64), die größer als die Breite (66) der Öffnung ist, erstreckt, und der Schlitz (34) wirkt in der geschlossenen Position in größerem Ausmaß auf die Bewegung des länglichen Glieds (56) entlang der Einführrichtung (58) ein als in der offenen Position.

2. Brille nach Anspruch 1, wobei das längliche Glied (56) durch die Öffnung (60) in der offenen Position, die senkrecht zu der geschlossenen Position liegt, einführbar ist und in der geschlossenen Position nicht durch die Öffnung (60) einführbar ist.

3. Brille nach Anspruch 2, ferner umfassend einen Über-Totpunkt-Mechanismus (36), der den Schaft (38) in Eingriff nimmt, wobei der Über-Totpunkt-Mechanismus (36) den Schaft (38) und das längliche Glied (56) von einer Zwischenposition zwischen der offenen und der geschlossenen Position weg vorspannt.

4. Brille nach Anspruch 2, ferner umfassend eine an dem länglichen Glied (56) befestigte Platte (74), wobei ein Abschnitt der Linse (14) zwischen der Platte (74) und der Fassung (12) positioniert ist, wenn das längliche Glied (56) in der geschlossenen Position in dem Schlitz (34) ist.

5. Brille nach Anspruch 1, wobei das längliche Glied (56) ein erstes und ein zweites Ende (67, 68) hat, wobei das zweite Ende (68) breiter als das erste Ende (67) ist.

6. Brille nach Anspruch 1, wobei die Linse (14) eine erste und eine zweite Linse (14a, 14b) aufweist, die aneinander befestigt sind, wobei der Schlitz (34) in einem Abschnitt der zweiten Linse (14b) ausgebildet ist, der sich von einem Rand der ersten Linse (14a) nach außen erstreckt.

7. Brille nach Anspruch 6, wobei der Rand der ersten Linse (14a) so bemessen ist, dass er in einen inneren Rand der Fassung (12) passt und wobei die zweite Linse (14b) eine Fläche der Fassung (12) in Eingriff nimmt, die sich von dem inneren Rand nach außen erstreckt.

8. Brille nach Anspruch 1, wobei die Brille eine Schutzbrille (10) ist.

9. Brille nach Anspruch 1, wobei die Brille eine Sonnenbrille ist.

10. Brille nach Anspruch 1, wobei der Schlitz (34) ein erster Schlitz ist, der in dem oberen Rand (16) der Linse (14) positioniert ist, und wobei das Befestigungselement (24) ein erstes Befestigungselement ist, das an dem oberen Abschnitt (20) der Fassung (12) montiert ist, wobei die Brille ferner einen zweiten Schlitz (34), der in dem unteren Rand (18) der Linse (14) positioniert ist, und ein zweites Befestigungselement (24) umfasst, das an dem unteren Abschnitt (22) der Fassung (12) montiert ist, wobei das zweite Befestigungselement (24) den zweiten Schlitz (34) gezielt in Eingriff nimmt, um die Linse (14) mit der Fassung (12) in Eingriff zu halten.

## Revendications

1. Lunettes (10), comprenant :
un verre (14) ayant un périmètre supérieur (16) et un périmètre inférieur (18), le verre définissant au moins une fente (34) formée dans l'un des périmètres supérieur et inférieur (16, 18) ;
un cadre (12) venant en prise de manière sélective avec un périmètre du verre (14), le cadre comprenant une portion supérieure (20) s'étendant le long du périmètre supérieur (16) et une portion inférieure (22) s'étendant le long du périmètre inférieur (18) ; et
un élément d'attache (24) monté de manière pivotante sur l'une des portions supérieure et inférieure (20, 22), l'élément d'attache (24) venant en prise de manière sélective avec l'au moins une fente (34) de manière à retenir le verre (14) en prise avec le cadre (12),
l'élément d'attache (24) comportant un organe allongé (56) monté sur une tige (38) montée de manière rotative sur le cadre (12) et dont la direction d'allongement est perpendiculaire à l'axe de rotation de la tige (38), l'organe allongé (56) pouvant tourner à l'intérieur de la fente (34) d'une position ouverte à une position fermée, dans la position ouverte, l'organe allongé (56) étant orienté par rapport à la fente (34) de telle sorte que l'organe allongé (56) soit configuré pour être inséré le long d'une direction d'insertion (58) à travers une ouverture (60) de la fente (34) qui s'étend depuis le périmètre du verre (14) jusqu'à la portion élargie (62) dont la largeur (64) est supérieure à la largeur (66) de l'ouverture, et dans la position fermée, la fente (34) interférant avec le mouvement de l'organe allongé (56) le long de la direction d'insertion (58) dans une plus grande mesure que dans la position ouverte.

2. Lunettes selon la revendication 1, l'organe allongé (56) pouvant être inséré à travers l'ouverture (60) dans la position ouverte perpendiculairement à la position fermée et ne pouvant pas être inséré à travers l'ouverture (60) dans la position fermée.

3. Lunettes selon la revendication 2, comprenant en outre un mécanisme de décentrage (36) venant en prise avec la tige (38), le mécanisme de décentrage (36) sollicitant la tige (38) et l'organe allongé (56) à l'écart d'une position intermédiaire entre les positions ouverte et fermée.

4. Lunettes selon la revendication 2, comprenant en outre une plaque (74) fixée à l'organe allongé (56), une partie du verre (14) étant positionnée entre la plaque (74) et le cadre (12) lorsque l'organe allongé (56) est dans la position fermée à l'intérieur de la fente (34).

5. Lunettes selon la revendication 1, l'organe allongé (56) ayant des première et deuxième extrémités (67, 68), la deuxième extrémité (68) étant plus large que la première extrémité (67).

6. Lunettes selon la revendication 1, le verre (14) comportant des premier et deuxième verres (14a, 14b) fixés l'un à l'autre, la fente (34) étant formée dans une portion du deuxième verre (14b) s'étendant vers l'extérieur depuis un périmètre du premier verre (14a).

7. Lunettes selon la revendication 6, le périmètre du premier verre (14a) étant dimensionné de manière à s'ajuster à l'intérieur d'un périmètre intérieur du cadre (12) et le deuxième verre (14b) venant en prise avec une face du cadre (12) s'étendant vers l'extérieur depuis le périmètre intérieur.

8. Lunettes selon la revendication 1, les lunettes étant des lunettes de protection (10).

9. Lunettes selon la revendication 1, les lunettes étant des lunettes de soleil.

10. Lunettes selon la revendication 1, la fente (34) étant une première fente positionnée dans le périmètre supérieur (16) du verre (14) et l'élément d'attache (24) étant un premier élément d'attache monté sur la portion supérieure (20) du cadre (12), les lunettes comprenant en outre une deuxième fente (34) positionnée dans le périmètre inférieur (18) du verre (14) et un deuxième élément d'attache (24) monté au niveau de la position inférieure (22) du cadre (12), le deuxième élément d'attache (24) venant en prise de manière sélective avec la deuxième fente (34) de manière à retenir le verre (14) en prise avec le cadre (12).
